# EUROPÄISCHE PATENTSCHRIFT

(11) **EP 2 803 336 B1**
(45) Veröffentlichungstag und Bekanntmachung des Hinweises auf die Patenterteilung: **08.03.2017**
(21) Anmeldenummer: 14168563.6
(22) Anmeldetag: 16.05.2014
(51) Int. Cl.: A61F 2/38, A61F 2/30

(54) **Vorrichtung zur in situ-Herstellung von artikulierenden Spacern**
Device for the in-situ production of articulating spacers
Dispositif de fabrication in situ de dispositifs d'espacement articulés

(30) Priorität: 17.05.2013 DE 102013209171
(43) Veröffentlichungstag der Anmeldung: 19.11.2014
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- US-A1- 2010 292 803

## Beschreibung

Die Erfindung betrifft eine Vorrichtung zur in-situ Herstellung von Gelenkspacern aus einem Knochenzement aufweisend eine Stempelfläche zur Ausformung einer Artikulationsoberfläche eines Spacers in dem Knochenzement, wobei der Knochenzement auf einer Knochenoberfläche zur Befestigung des Spacers angeordnet ist, wobei die Stempelfläche ein Negativ der zu erzeugenden Artikulationsoberfläche aufweist. Die Erfindung betrifft auch ein Set umfassend zwei solcher Vorrichtungen, die Verwendung einer solchen Vorrichtung und ein Verfahren zur Herstellung eines Gelenkspacers auf einem Knochen unter Verwendung einer solchen Vorrichtung. Die Vorrichtungen sind insbesondere zur Herstellung von Kniegelenkspacern geeignet.

Die mit erfindungsgemäßen Vorrichtungen hergestellten Kniegelenkspacer werden während einer Operation (OP) in situ direkt an der proximalen Tibia und dem distalen Femur unter Verwendung von PMMA-Knochenzementteig (Polymethylmethacrylat-Knochenzementteig) und der Vorrichtung gebildet, ohne dass separate Herstellungsschritte für die Kniespacerkomponenten und deren nachfolgende Verankerung an der proximalen Tibia und dem distalen Femur notwendig sind.

Gelenkendoprothesen haben gegenwärtig eine Standzeit von mehreren Jahren, zum Beispiel bei zementierten Hüftgelenkprothesen im Durchschnitt grösser als zehn bis fünfzehn Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen. Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich mit sehr hohen Kosten verbunden ist. Sowohl bei der aseptischen als auch der septischen Lockerung wird üblicherweise eine Revision vorgenommen. Man unterscheidet einzeitige und die zweizeitige Revisionen. Bei septischen Lockerungen werden sehr häufig zweizeitige Revisionen vorgenommen.

Bei der zweizeitigen Revision wird in einer ersten OP die infizierte Gelenkendoprothese entfernt, es erfolgt ein Debridement und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen. Man unterscheidet dann nichtartikulierende und artikulierende Spacer. Artikulierende Spacer oder Gelenkspacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Gelenkspacer stellen heute den Stand der Technik dar. Der Spacer wird in einer zweiten OP entfernt, es wird nochmals debridiert und dann wird eine zementierte oder auch zementfreie Revisions-Gelenkendoprothese implantiert.

Die Verwendung von Spacern geht ursprünglich auf Hovelius und Josefsson zurück (Hovelius L, Josefsson G (1979), "An alternative method for exchange Operation of infected arthroplasty", Acta Orthop. Scand. 50: 93-96). Weitere frühe Arbeiten zu Spacern stammen von Younger (Younger AS, Duncan CP, Masri BA, McGraw RW (1997), "The outcome of two-stage arthroplasty using a custom-made interval spacer to treat the infected hip", J. Arthroplasty 12: 615-623), Jones (Jones WA, Wroblewski BM (1989), "Salvage of failed total knee arthroplasty: the 'beefburger' procedure", J. Bone Joint Surg. Br. 71: 856-857.) und Cohen (Cohen JC, Hozack WJ, Cuckler JM, Booth RE Jr (1988), "Two-stage reimplantation of septic total knee arthroplasty, Report of three cases using an antibiotic-PMMA spacer block", J. Arthroplasty 3: 369-377). Von McPherson stammt die Konzeption, dass Spacer ausschließlich aus Knochenzement hergestellt werden können (McPherson EJ, Lewonowski K, Dorr LD (1995), "Techniques in arthroplasty. Use of an articulated PMMA spacer in the infected total knee arthroplasty", J. Arthroplasty 10: 87-89).

Es gibt auf dem Markt mit Antibiotika ausgerüstete Spacer zum temporären Ersatz von Knie-, Hüft- und Schultergelenkendoprothesen. Nachteilig daran ist jedoch, dass die enthaltenen Antibiotika vorgegeben sind und nicht entsprechend dem Antibiogramm der vorgefundenen mikrobiellen Keime speziell angepasst werden können. Im Fall der Kniespacer müssen zusätzlich sowohl die Tibia- als auch die Femurkomponente mit PMMA-Knochenzement an der proximalen Tibia und am distalen Femur verankert werden.

Spacer werden häufig vom Chirurgen aus konventionellen PMMA-Knochenzementen unter Nutzung geeigneter Gießformen selbst hergestellt. Dabei werden dem PMMA-Knochenzement-Pulver vor der Spacer-Herstellung entsprechend den bei zuvor durchgeführten Punktionen nachgewiesenen mikrobiellen Keimen und nach erfolgtem Antibiogramm ein oder mehrere Antibiotika zugemischt. Die Antibiotika-Auswahl wird speziell auf die vorliegenden mikrobiellen Keime abgestimmt. Dieses Vorgehen ist insbesondere beim Auftreten von mehrfach resistenten Keimen oder bei Mischinfektionen mit unterschiedlichen Keimen sehr vorteilhaft. Die mit Gießformen hergestellten Spacer müssen, insbesondere bei Hüftspacern, zur Entfernung von Graten mechanisch nachgearbeitet werden. Bei Kniespacern müssen sowohl die Tibia- als auch die Femurkomponente des Spacers mit PMMA-Knochenzementteig anschließend an der proximalen Tibia beziehungsweise am distalen Femur fixiert werden. Das bedeutet, es ist ein erheblicher Zeit- und Arbeitsaufwand zur Herstellung von Spacern mit Gießformen sowie deren nachfolgenden Implantation notwendig.

In der Praxis ist es daher bei der zweizeitigen Revision von Kniegelenkendoprothesen auch üblich, nach Entfernung der primären Gelenkendoprothese und nach erfolgtem Debridement PMMA-Knochenzementteig auf die proximale Tibia und den distalen Femur aufzutragen und dort manuell Gleitflächen zu formen.

Dieses interessante Konzept wurde von der US 2004/0036189 A1 aufgenommen. Dort wird ein Gießformsystem für Kniespacer vorgeschlagen, das dadurch charakterisiert ist, dass die Gießformen zur in situ-Formung der Spacer-Komponenten an der proximalen Tibia und dem distalen Femur vorgenommen wird. Die vorgeschlagenen elastischen Gießformen werden nach Befüllung mit PMMA-Zementteig direkt an den Knochen angebracht und nach Aushärtung des Zementteigs wieder entfernt. Das bedeutet, dass die Spacerkomponenten in einem Schritt gleichzeitig geformt, mit dem Knochengewebe verbunden und ausgehärtet werden. Dadurch kann sehr viel Zeit- und Arbeitsaufwand gespart werden. Die Gießformen besitzen neben der Konturfläche für die Formung der Gleitflächen einen Seitenrand, der die Konturflächen umgibt und dadurch einen Raum bildet, in dem Spacer geformt wird und ausgehärtet wird.

Nachteilig ist an diesem Gießformsystem jedoch, dass die eingeschlossene Luft nicht entweicht und dass Lufteinschlüsse an den Artikulationsflächen beziehungsweise Gleitflächen der Gelenkspacer Schäden verursachen können und dadurch die Gleiteigenschaften des artikulierenden Spacers beeinträchtigen können. Weiterhin ist es problematisch, die Gießformen nach Aushärtung des PMMA-Zementteigs vom ausgehärteten Spacer abzutrennen, ohne dabei die Spacer und insbesondere die Artikulationsflächen beziehungsweise Gleitflächen zu beschädigen oder zu beeinträchtigen. Zudem ist der Aufbau der Gießformen komplex. Dadurch, dass die Gießformen an dem Knochen anliegen müssen, muss eine Vielzahl verschiedener Gießformen bereitgehalten werden oder diese müssen sogar individuell angefertigt werden. Die Anwendung während einer OP könnte zudem einfacher und störungsunanfälliger sein.

US 2010/0292803 A1 beschreibt eine Form und ein Verfahren zur Herstellung eines Gelenkspacers in-situ, wobei die Form auf einem Knochenteil eines Patienten angeordnet wird, welche so ausgestaltet ist, so dass sich dass zu formende biokompatible Material optimal der Anatomie des Patienten anpasst.

Die Aufgabe der Erfindung besteht also darin, die Nachteile des Stands der Technik zu überwinden. Insbesondere soll die Vorrichtung und das Verfahren eine Möglichkeit bereitstellen, auf einfache Weise eine möglichst ebene und glatte Artikulationsoberfläche zu erzeugen, die eine Bewegung des erzeugten Spacers ermöglicht, ohne dass es zu einer

Verschlechterung der Beweglichkeit des Gelenks oder zu Schmerzen beim Patienten aufgrund eines Abriebs an den artikulierenden Gleitflächen des Spacers kommt. Die Vorrichtung und das Verfahren sollen möglichst universell einsetzbar sein.

Aufgabe der Erfindung ist es auch, eine Vorrichtung zur in-situ-Herstellung von artikulierenden Kniespacern zu entwickeln, welche die Nachteile der bisherigen Vorrichtungen zur Herstellung von Kniespacern überwindet. Die Vorrichtung soll eine schnelle in-situ-Formgebung der Gleitflächen ermöglichen, wobei die Gefahr des Einschlusses von Luftblasen weitgehend vermieden werden soll. Außerdem soll die Vorrichtung eine leichte Abtrennung vom ausgehärteten Spacer ermöglichen, ohne dass Schäden an den Gleitflächen auftreten. Die Vorrichtung soll einfach und kostengünstig aus technisch üblichen Kunststoffen zu fertigen sein. Die Aufgaben der Erfindung werden gelöst durch eine Vorrichtung zur in-situ Herstellung von Gelenkspacern aus einem Knochenzement aufweisend eine Stempelfläche zur Ausformung einer Artikulationsoberfläche eines Spacers in dem Knochenzement, wobei der Knochenzement auf einer Knochenoberfläche zur Befestigung des Spacers angeordnet ist, wobei die Stempelfläche ein Negativ der zu erzeugenden Artikulationsoberfläche aufweist und die das Negativ bildende Oberfläche zumindest bereichsweise seitlich offen ist, so dass der Knochenzement von der zu formenden Artikulationsoberfläche seitlich wegdrückbar ist, wobei auf der Stempelfläche zumindest ein Abstandhalter zur Auflage auf dem Knochen angeordnet ist, insbesondere zur Auflage auf der Knochenoberfläche angeordnet ist, wobei mit dem Abstandhalter die Dicke des Knochenzements auf der Knochenoberfläche einstellbar ist.

Dabei ist hier und im Folgenden mit der Knochenoberfläche die Oberfläche des Knochens gemeint, auf der der Knochenzement zur Bildung des Gelenkspacers aufgebracht wird beziehungsweise aufgebracht ist.

Durch die seitliche Öffnung können Lufteinschlüsse vermieden werden, da sie durch die seitlichen Öffnungen herausgedrückt werden.

Bei erfindungsgemäßen Vorrichtungen kann auch vorgesehen sein, dass die das Negativ bildende Oberfläche im Wesentlichen oder vollumfänglich seitlich offen ist, so dass der Knochenzement von der zu formenden Artikulationsoberfläche seitlich in alle Richtungen wegdrückbar ist.

Bevorzugt ist die das Negativ bildende Oberfläche zu zumindest 70% seitlich offen, besonders bevorzugt zu zumindest 90% seitlich offen, ganz besonders bevorzugt vollumfänglich seitlich offen.

Seitlich offen bedeutet in diesem Zusammenhang, dass neben der das Negativ bildende Oberfläche keine in Richtung der Oberfläche geneigte Fläche angrenzt, die einen Hohlraum zwischen der Vorrichtung und dem zu formenden Knochenzement bildet, wenn die Vorrichtung zum bestimmungsgemäßen Gebrauch auf den Knochenzement gedrückt wird.

Das hat den Vorteil, dass sich möglichst wenige oder bevorzugt keine solchen Hohlräume beim Stempeln beziehungsweise beim Andrücken der Vorrichtung zwischen dem Knochenzement und der Vorrichtung bilden können, die dann anschließend zur Bildung von Hohlräumen in der Artikulationsoberfläche führen können, die die Stabilität der Artikulationsoberfläche beeinträchtigen würden.

Die Höhe des Abstandhalters beziehungsweise der Abstandhalter bezogen auf die Stempelfläche gibt beziehungsweise geben automatisch die Dicke des Knochenzements vor, wenn der oder die Abstandhalter auf den Knochen beziehungsweise die Knochenoberfläche gepresst werden. Die Dicke des erzeugten Spacers ist so auf einfache Weise definiert. Die Vorrichtung wird dadurch auch im hektischen Operationsbetrieb sehr einfach Anwendbar.

Mit einer erfindungsgemäßen Weiterbildung der Erfindung wird vorgeschlagen, dass auf der Stempelfläche zumindest zwei Abstandhalter zur Auflage auf dem Knochen angeordnet sind, insbesondere auf der Knochenoberfläche angeordnet ist, mit denen die Dicke des Knochenzements auf der Knochenoberfläche einstellbar ist. Es kann erfindungsgemäß auch vorgesehen sein, dass auf der Stempelfläche drei triangulär zueinander angeordnete Abstandhalter zur Auflage auf dem Knochen angeordnet sind.

Die Abstandhalter bewirken auf einfache Weise, dass der Knochenzement mit einer gewünschten Stärke beziehungsweise Dicke aufgebracht werden kann, ohne dass sich an der Artikulationsfläche Lufteinschlüsse bilden können. Bevorzugt ist der Abstandhalter beziehungsweise sind die Abstandhalter konisch in Richtung von der Stempelfläche weg zusammenlaufend ausgebildet. Ebenfalls bevorzugt kann ferner vorgesehen sein, dass der Abstandhalter oder die Abstandhalter im Inneren der Stempelfläche und nicht randseitig angeordnet ist oder sind.

Es wird ferner vorgeschlagen, dass die Höhe der Abstandhalter oder des Abstandhalters über der Stempelfläche variabel einstellbar ist.

Dadurch können verschiedene Zementteigdicken erzeugt werden, beziehungsweise die Stärke des Spacers variabel erzeugt werden.

Gemäß einer bevorzugten Weiterbildung der Erfindung, kann auch vorgesehen sein, dass der Abstandhalter oder die Abstandhalter einen durchdrückbaren Boden als Auflage auf dem Kochen, insbesondere der Knochenoberfläche aufweist oder aufweisen, wobei sich der durchdrückbare Boden unter Einwirkung einer Kraft in Richtung des Knochens, insbesondere der Knochenoberfläche ausbeult und dadurch die Stempelfläche von dem mit der Vorrichtung geformten Knochenzement ablösbar ist, wobei der Boden bevorzugt Gummielastisch und die restliche Vorrichtung nicht Gummi-elastisch ist.

Die Vorrichtung ist dann besonders leicht und einfach vom ausgehärteten Zementteig abzulösen. Durch das Durchwölben des Bodens der Abstandhalter hebt sich der Rest der Vorrichtung von dem Knochen beziehungsweise der Knochenoberfläche ab.

Mit einer bevorzugten Ausführungsform der vorliegenden Erfindung wird vorgeschlagen, dass die Vorrichtung zumindest eine Ausstoßeinrichtung aufweist, mit der die Stempelfläche von dem mit der Vorrichtung geformten Knochenzement durch Einwirken einer Kraft mechanisch ablösbar ist, wobei bevorzugt die Ausstoßeinrichtung in dem Abstandhalter gelagert ist oder die Ausstoßeinrichtungen in den Abstandhaltern gelagert sind.

Dies Vereinfacht das Ablösen der Vorrichtung vom zumindest weitgehend ausgehärteten Zementteig erheblich. Zudem kann durch die definierte Krafteinwirkung sichergestellt werden, dass es zu keiner mechanischen Belastung der geformten Spacer kommt, die zu einer Beschädigung derselben führen könnte.

Dabei kann vorgesehen sein, dass die Ausstoßeinrichtung ein axial beweglicher Stift oder Stößel ist oder eine über ein Gewinde mit der Vorrichtung verbundene Schraube ist, mit dem oder mit der die Vorrichtung auf der Stempelflächenseite vom Knochen, insbesondere von der Knochenoberfläche wegdrückbar ist, wobei bevorzugt die Stifte, die Stößel und/oder Schrauben mehrerer Ausstoßeinrichtungen derart miteinander verbunden sind, dass sie sich nur alle gemeinsam auf den Knochen, insbesondere die Knochenoberfläche drücken lassen.

Mit diesen Ausstoßeinrichtungen sind besonders einfach gut zu dosierende und wohl gerichtete Kraftmomente auf die Vorrichtung ausübbar.

Ferner kann dabei vorgesehen sein, dass die Kraft der Ausstoßeinrichtung senkrecht zur Stempelfläche auf den Knochen, insbesondere auf die Knochenoberfläche wirkt.

Bei Ausführungsformen mit Ausstoßeinrichtungen kann zudem vorgesehen sein, dass die Ausstoßeinrichtung in einer Öffnung senkrecht zur Stempelfläche angeordnet ist oder die Ausstoßeinrichtungen in Öffnungen senkrecht zur Stempelfläche angeordnet sind.

Durch diese Maßnahmen werden Torsionskräfte und Drehmomente vermieden, die beim Ablösen der Vorrichtung zu einer Beschädigung des Spacers führen könnten.

Mit einer Weiterentwicklung der Erfindung wird vorgeschlagen, dass an der von der Stempelfläche abgewandten Seite und/oder an der an die Stempelfläche angrenzenden Seitenfläche der Vorrichtung zumindest ein Griff zum Anpressen der Vorrichtung an den Knochen angeordnet ist.

Dies Vereinfacht die Bedienung der Vorrichtung und hilft insbesondere auch bei der Verwendung der Ausstoßeinrichtung.

Zum Aufbau erfindungsgemäßer Vorrichtungen kann vorgesehen sein, dass die Vorrichtung im Wesentlichen aus einem thermoplastischen Kunststoff gefertigt ist, bevorzugt aus Polypropylen, Polyethylen, Polytetrafluorethylen, Polyethylenterephthalat, Polybutylenterephathalat, Polyamid-6, Polyamid-6.6, Polyamid-10, Polyamid-12 oder deren Gemischen besteht, vorzugsweise die Vorrichtung gegebenenfalls bis auf den durchdrückbaren Boden, Stifte, Stößel und/oder Schrauben aus den genannten Materialien besteht.

Diese Materialien sind kostengünstig und sind gut zur Herstellung der Vorrichtungen geeignet. Zudem lassen sie sich gut desinfizieren und sind somit besonders gut zum Aufbau von Stempeln im medizinischen Bereich geeignet.

Auch kann vorgesehen sein, dass die Vorrichtung ein Stempel ist, bevorzugt ein Tibia-Stempel zum Formen eines Tibiaplateaus und/oder ein Femur-Stempel zum Formen einer Kondylenoberfläche ist.

Dabei kann vorgesehen sein, dass die Ausstoßeinrichtungen des Tibia-Stempels asymmetrisch zu einer zur Sagitalebene des Patienten parallelen Ebene im Knie angeordnet sind und/oder die Ausstoßeinrichtung oder die Ausstoßeinrichtungen des Femur-Stempels zwischen den Formen für die auszuformenden Kondylen angeordnet ist oder sind, bevorzugt axial zur Femurachse ausgerichtet ist oder sind.

Die Aufgaben der Erfindung werden auch gelöst durch ein Set umfassend zwei Vorrichtungen nach einem der vorangehenden Ansprüche, wobei die erste Vorrichtung zur Ausbildung der Artikulationsoberfläche eines ersten Gelenkspacers vorgesehen ist und die zweite Vorrichtung zur Ausbildung der Artikulationsoberfläche eines zweiten Gelenkspacers vorgesehen ist, wobei die beiden Artikulationsoberflächen im beim Patienten eingesetzten Zustand aneinander anliegen und die Gleitflächen des Gelenkspacers bilden, so dass der Gelenkspacer die Funktion der Knochen des betroffenen Gelenks übernimmt, bevorzugt eines Kniegelenks übernimmt.

Das Set hat den Vorteil, dass die Vorrichtungen des Sets gleich die zueinander passenden Stempelformen haben, so dass die Artikulationsflächen der erzeugten Spacer beziehungsweise Spacerteile aufeinander abgestimmt sind.

Die Aufgaben der Erfindung werden ferner gelöst durch die Verwendung einer solchen Vorrichtung oder eines solchen Sets zur in-situ-Formung zumindest eines Gelenkspacers, bevorzugt zur in-situ-Formung eines Tibia-Spacers und/oder Femur-Spacers.

Auch werden die der Erfindung zugrunde liegenden Aufgaben gelöst durch ein Verfahren zur Herstellung eines Gelenkspacers auf einem Knochen, insbesondere der Knochenoberfläche, aufweisend die folgenden chronologischen Verfahrensschritte:
A) Aufbringen eines Knochenzements auf einen vorbereiteten Knochen;
B) Aufdrücken einer erfindungsgemäßen Vorrichtung; und
C) Lösen der Vorrichtung von dem gehärteten Knochenzement.

Der Knochenzement muss in Schritt C) noch nicht völlig ausgehärtet sein, darf aber nicht mehr eine teigige Konsistenz aufweisen. Dennoch ist es erfindungsgemäß bevorzugt, wenn der Knochenzement zum Lösen der Vorrichtung vollständig ausgehärtet ist.

Dabei kann vorgesehen sein, dass nach Schritt A) und vor Schritt B) der Knochenzement aushärtet, bis er eine teigige Konsistenz annimmt. Eine teigige Konsistenz ist erreicht, wenn der Knochenzement derartig zähflüssig ist, dass er noch formbar ist, ohne dass die Oberflächenspannung des Knochenzements zu einer Verformung der Oberfläche des Knochenzements führt.

Ferner kann erfindungsgemäße bevorzugt vorgesehen sein, dass bei Schritt C) die Vorrichtung durch Verwendung der Ausstoßeinrichtung von der geformten Knochenzementoberfläche abgelöst wird, bevorzugt durch Schrauben zumindest einer Schraube als Ausstoßeinrichtung und/oder durch Drücken auf zumindest einen Stift oder zumindest einen Stößel als Ausstoßeinrichtung und/oder durch Durchdrücken des Bodens der Abstandhalter, insbesondere mit zumindest einem Stift oder zumindest einen Stößel oder zumindest einer Schraube als Ausstoßeinrichtung.

Es wird auch vorgeschlagen, dass bei Schritt B) die Vorrichtung derart auf den Knochenzement aufgedrückt wird, dass der Abstandhalter oder die Abstandhalter auf dem Knochen, insbesondere der Knochenoberfläche aufliegt oder aufliegen.

Schließlich kann auch vorgesehen sein, dass nach Schritt C) überschüssiger Knochenzement, der seitlich über die Artikulationsoberfläche hinausragt, entfernt wird.

Der Erfindung liegt die überraschende Erkenntnis zugrunde, dass es durch die offenen Seitenflächen gelingt, einen Stempel bereitzustellen, der keine oder nur wenige Hohlraumbildende Seitenwände aufweist, so dass sich keine oder kaum Lufteinschlüsse auf der zu erzeugenden Artikulationsoberfläche des Spacers bilden können, da die Lufteinschlüsse seitlich mit dem überschüssigen Zement herausgedrückt werden. Dadurch wird die erzeugte artikulierende Oberfläche des Spacers glatter und es wird die Gefahr reduziert, dass sich Partikel aus der Oberfläche der Gleitfläche lösen und anschließend zu Komplikationen beim Patienten führen.

Die Erfindung beruht unter anderem auch auf der Erkenntnis, dass ein teigiger (zähflüssiger) Knochenzement auch problemlos ohne vollständig umschließende Gießform geformt werden kann, wenn er nicht zu flüssig ist. Die geeignete teigige Konsistenz kann erreicht werden, indem nach dem Aufbringen des Knochenzements auf den Knochen eine gewisse Zeit abgewartet wird, bis der Zement beginnt auszuhärten, beziehungsweise zähflüssig zu werden, oder indem gleich eine Knochenzementmischung mit der gewünschten Konsistenz hergestellt wird. Eine solche seitlich nicht oder nur bereichsweise begrenzte Vorrichtung muss nicht umfänglich am Knochen anliegen, so dass die Vorrichtung variabel für eine Vielzahl verschiedener Patienten beziehungsweise verschiedener Knochen-Geometrien einsetzbar ist.

Der Erfindung liegt die Idee zu Grunde, Stempel zur Formgebung der Artikulationsflächen beziehungsweise Gleitflächen zu nutzen, wobei die Stempel bevorzugt mindestens einen beweglichen Ausstoßer enthalten, mit dem die Stempel von dem ausgehärteten PMMA-Zement losgelöst werden können. Das bedeutet, der medizinische Anwender bestreicht beispielsweise die proximale Tibia und/oder den distalen Femur mit einem, mit geeigneten Antibiotika modifiziertem PMMA-Zement. Dann wird die tibiale Artikulationsfläche beziehungsweise Gleitfläche durch Aufpressen eines Tibia-Stempels geformt. Die Dicke des Tibia-Spacers wird durch den Abstand der Auflagefläche des Ausstoßers zur Kontur des Stempels zuvor bestimmt. Die Entfernung des Stempels kann entweder erfolgen, wenn der PMMA-Zementteig nicht mehr fließfähig ist, oder wenn der PMMA-Zement bereits ausgehärtet ist. In gleicher Weise funktioniert der Femur-Stempel, wobei die Artikulationsflächen beziehungsweise Gleitflächen der Kondylen durch den Femur-Stempel geformt werden. Die nachfolgende Vorgehensweise gleicht der geschilderten Vorgehensweise beim Tibia-Stempel.

Wesentlich ist, dass die Vorrichtung, also beispielsweise der Tibia-Stempel oder der Femur-Stempel, keine Umrandung aufweist, die zusammen mit der Konturfläche einen Hohlraum bilden könnte. Dadurch ist es möglich, dass überschüssiger PMMA-Zement an den Rändern entweichen kann und dass weiterhin Lufteinschlüsse problemlos aus dem Zementteig austreten können. Dadurch werden durch Lufteinschlüsse verursachte Fehler der Artikulationsflächen beziehungsweise Gleitflächen wirksam vermieden. Der überschüssige Zementteig kann währenddessen oder anschließend problemlos manuell oder mit Hilfe von Spateln entfernt werden. Entscheidend ist, dass die so erzeugten Artikulationsflächen eine glatte ungestörte Oberfläche beziehungsweise Artikulationsfläche besitzen.

Erfindungsgemäß ist beispielsweise eine Vorrichtung zur Herstellung von Kniegelenkspacern unter Verwendung von PMMA-Knochenzementen, die dadurch charakterisiert ist, dass die Vorrichtung aus zwei Komponenten A und B besteht, wobei die Komponente A aus
a) einem Tibia-Stempel (erste Vorrichtung) besteht, dessen Unterseite eine zum formenden Tibiaplateau (Artikulationsfläche der Tibia) negative Kontur aufweist,
b) wobei der Stempel mindestens eine senkrecht zur Kontur durchgehende Öffnung aufweist,
c) wobei mindestens ein verschiebbarer Ausstoßer (Ausstoßeinrichtung) vorgesehen ist, der durch die mindestens eine Öffnung des Stempels senkrecht zur Kontur verschiebbar ist,
d) wobei der Stempel an seiner Oberseite mindestens ein Griffstück aufweist und
e) wobei der Tibia-Stempel keine einen Hohlraum formenden Seitenwände besitzt, welche die Kontur bildende Oberfläche umgrenzen,
und die Komponente B aus
a) einem Femur-Stempel (zweite Vorrichtung) besteht, dessen Unterseite eine zu den formenden Kondylen (Artikulationsflächen des Femur) negative Kontur aufweist,
b) wobei der Stempel mindestens ein senkrecht zur Kontur durchgehende Öffnung aufweist,
c) wobei mindestens ein verschiebbarer Ausstoßer (Ausstoßeinrichtung) vorgesehen ist, der durch die mindestens eine Öffnung des Stempels senkrecht zur Kontur verschiebbar ist,
d) wobei der Femur-Stempel an seiner Oberseite mindestens ein Griffstück aufweist und
e) wobei der Femur-Stempel keine einen Hohlraum formenden Seitenwände besitzt, welche die Kontur bildende Oberfläche umgrenzen.

Erfindungsgemäß kann auch vorgesehen sein, dass der mindestens eine Ausstoßer des Tibia-Stempels und/oder des Femur-Stempels durch manuelle lineare Verschiebung oder durch Schraubbewegungen senkrecht zur formenden Kontur bewegt werden kann.

Ferner kann vorgesehen sein, dass der mindestens eine Ausstoßer mindestens eine Auflagefläche besitzt, die sich auf dem Knochen der Tibia/ des Femurs abstützt, wobei die Auflagefläche des Ausstoßers bevorzugt gummielastisch ist.

Eine bevorzugte und vorteilhafte Ausgestaltung besteht darin, dass der Ausstoßer graduiert ist und dass der Abstand zwischen der Auflagefläche des Ausstoßers und der Kontur des Tibia-Stempels/des Femur-Stempels festgelegt werden kann.

Erfindungsgemäß ist, dass der Tibia-Stempel/der Femur-Stempel, mit Ausnahme der Auflagefläche des Ausstoßers, aus einem nicht gummielastischen Material gefertigt ist.

Die erfindungsgemäße Komponente A wird zur in-situ-Formung von Tibia-Spacern verwendet und die erfindungsgemäße Komponente B zur in-situ-Formung von Femur-Spacern.

Im Folgenden werden Ausführungsbeispiele der Erfindung anhand von sechs schematisch dargestellten Figuren erläutert, ohne jedoch dabei die Erfindung zu beschränken. Dabei zeigt:
Figur 1: eine schematische perspektivische Ansicht eines erfindungsgemäßen Femur-Stempels;
Figur 2: eine schematische Aufsicht auf einen erfindungsgemäßen Tibia-Stempel;
Figur 3: eine schematische Querschnittansicht durch den Tibia-Stempel nach Figur 2;
Figuren 4 und 5: eine schematische Seitenansicht des Tibia-Stempels nach den Figuren 2 und 3 in einer Anwendungssituation; und
Figur 6: eine schematische Querschnittansicht eines erfindungsgemäßen Femur-Stempels.

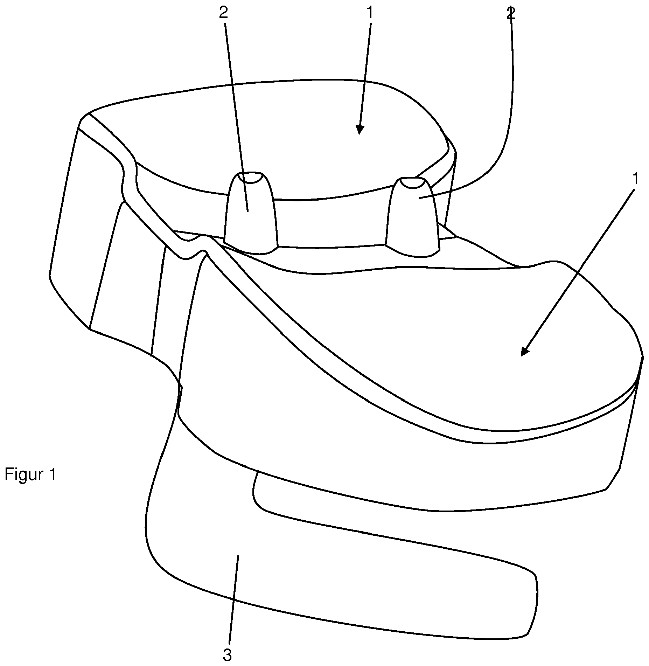

Figur 1 zeigt eine schematische perspektivische Ansicht eines erfindungsgemäßen Femur-Stempels, der aus einem thermoplastischen Kunststoff gefertigt ist. Der Femur-Stempel weist auf der in Figur 1 gezeigten Darstellung in Richtung nach oben eine Stempelfläche 1 auf, die zur Formung einer Artikulationsfläche in einem Knochenzement (nicht gezeigt) vorgesehen ist, wobei der Knochenzement auf einem vorbereiteten Ende eines Femur angeordnet ist. Der Knochenzement haftet dazu an der Auflagefläche auf dem FemurKnochen. Wenn die Ausgangs-Komponenten des Knochenzements miteinander reagieren, wird der Knochenzement zunächst teigig, das heißt zähflüssiger. In diesem Zustand wird der Femur-Stempel mit der Stempelfläche 1 auf den Knochenzement gepresst. Der fertig ausgehärtete Knochenzement bildet später einen Femur-Gelenk-Spacer und bildet zumindest die Kondylen des Femur nach.

Die Dicke des Knochenzements und damit später des Spacers wird durch die Höhe von zwei, auf einer Mittelebene des Femur-Stempels angeordneten Abstandhalter 2 bestimmt. Die abgeflachten Enden der Abstandhalter 2 dienen als Auflageflächen auf dem Femur-Kochen in dem Bereich, auf dem auch der Knochenzement aufgetragen ist. In dieser Mittelebene ist die Stempelfläche 1 leicht nach innen gewölbt. Die beiden äußeren, nach Innen gewölbten Bereiche der Stempelfläche 1 sollen die Gleitflächen der Kondylen des Spacers im Konchenzement formen.

Beim Aufpressen des Femur-Stempels auf den Knochenzement kann der Knochenzement allseitig über die Stempelfläche 1 hinaus abfließen, da der Femur-Stempel keine Seitenwände aufweist, die über die Kanten der Stempelfläche 1 hinaus ragen würden. Dadurch kann sichergestellt werden, dass die durch die Stempelfläche 1 erzeugte Artikulationsfläche oder Gleitfläche des aus dem Knochenzement erzeugten Spacers glatt ist und keine Löcher aufweist, die durch Lufteinschlüsse zwischen der Stempelfläche 1 und dem Knochenzement verursacht würden.

Auf der der Stempelfläche 1 gegenüberliegenden Seite des Femur-Stempels ist ein Griff 3 vorgesehen, mit dem der Femur-Stempel manuell gehalten und bedient werden kann.

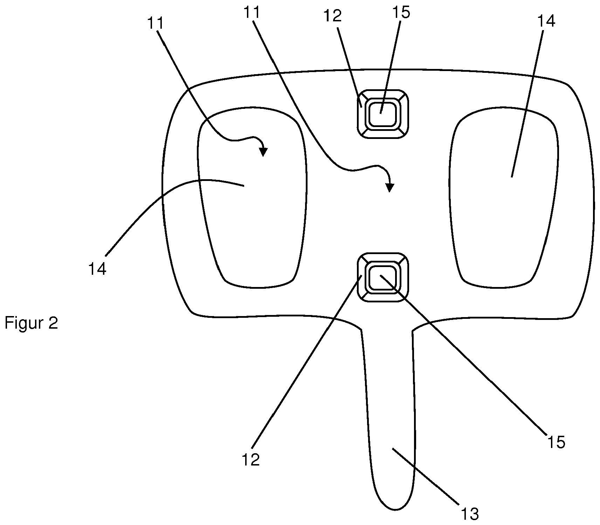

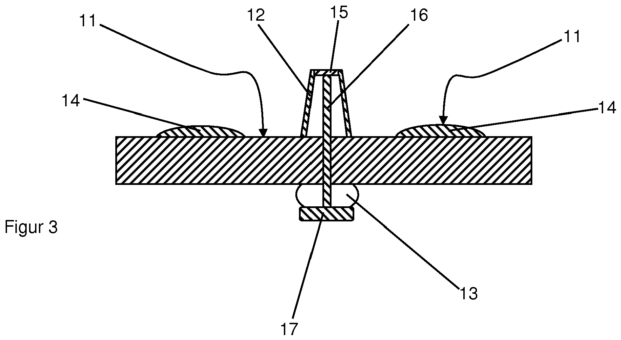

Figuren 2 und 3 zeigen eine schematische Aufsicht auf eine Stempelfläche 11 eines erfindungsgemäßen Tibia-Stempels, der im Wesentlichen aus einen harten KunststoffMaterial wie beispielsweise aus Polypropylen, Polyethylen und/oder einem Polyamid besteht. Figur 3 zeigt eine schematische Querschnittansicht durch den Tibia-Stempel nach Figur 2.

In der Mitte der Stempelfläche sind zwei konische Abstandhalter 12 angeordnet, die sich über die Stempelfläche 11 erheben. Entlang einer Mittelachse des Tibia-Stempels, die die Zentren der beiden Abstandhalter 12 verbindet, ist auf einer Seite des Tibia-Stempels ein Griff 13 angeordnet, mit dem der Tibia-Stempel auf einen Knochenzement pressbar ist.

Die Stempelfläche 11 ist durch zwei Laufflächenformen 14 strukturiert, die sich aus der Ebene Stempelfläche 11 erheben (in Figur 2 in Richtung des Betrachters).

Die ebenen Bodenflächen 15 der Abstandhalter 12, die als Auflageflächen auf dem Knochen dienen, sind im Zentrum aus einem gummielastischen oder einem geeignet vorgespannten Material gefertigt. Dadurch können die Bodenflächen 15 in Richtung des Knochens durchgewölbt werden.

Im Inneren der Abstandhalter sind Stifte 16 beziehungsweise Stößel 16 angeordnet, die entlang ihrer Mittelachse beweglich in Öffnungen in dem Tibia-Stempel gelagert sind. Die Stifte 16 beziehungsweise Stößel 16 ragen bis über die, der Stempelfläche 11 gegenüberliegende Seite des Tibia-Stempels hinaus. Dort sind sie mit einer Betätigungsleiste 17 verbunden, die die beiden Stifte 16 beziehungsweise Stößel 16 miteinander starr verbindet. Durch einen Druck auf die Betätigungsleiste 17 drücken die Stifte 16 beziehungsweise Stößel 16 auf die Innenseite der Bodenflächen 15 und wölben diese dadurch nach außen.

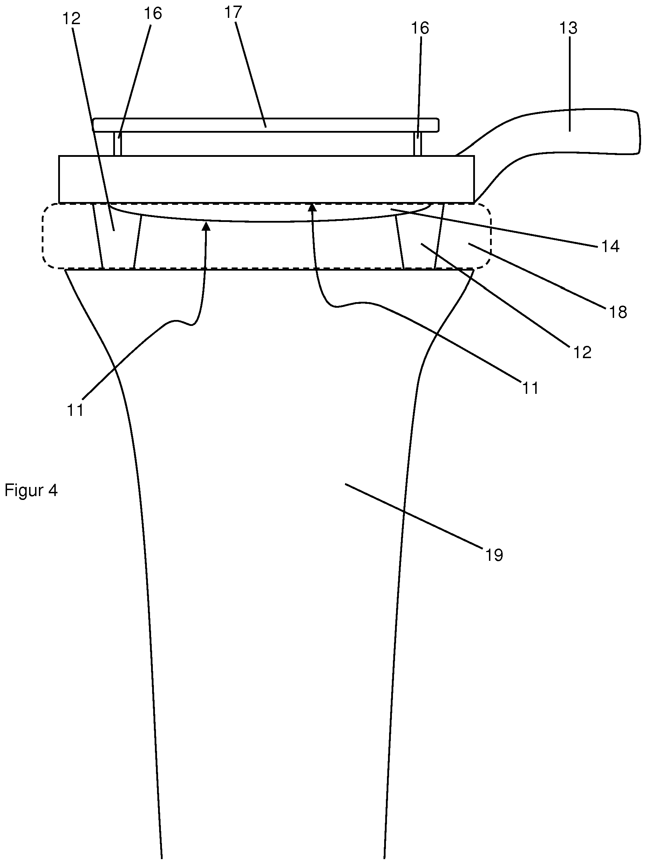

In Figur 4 ist eine schematische Seitenansicht des Tibia-Stempels nach den Figuren 2 und 3 in einer Anwendungssituation gezeigt. Der Tibia-Stempel ist auf einen Knochenzement 18 und auf ein Ende eines Tibia-Knochens 19 gepresst dargestellt. Um die Anordnung der Abstandhalter 12 und der Stempelfläche 11 zu verdeutlichen, ist der Knochenzement 18 in Figur 18XW transparent gezeigt und daher nur als rechteckige Form mit abgerundeten Ecken durch eine gestrichelte Linie kenntlich gemacht.

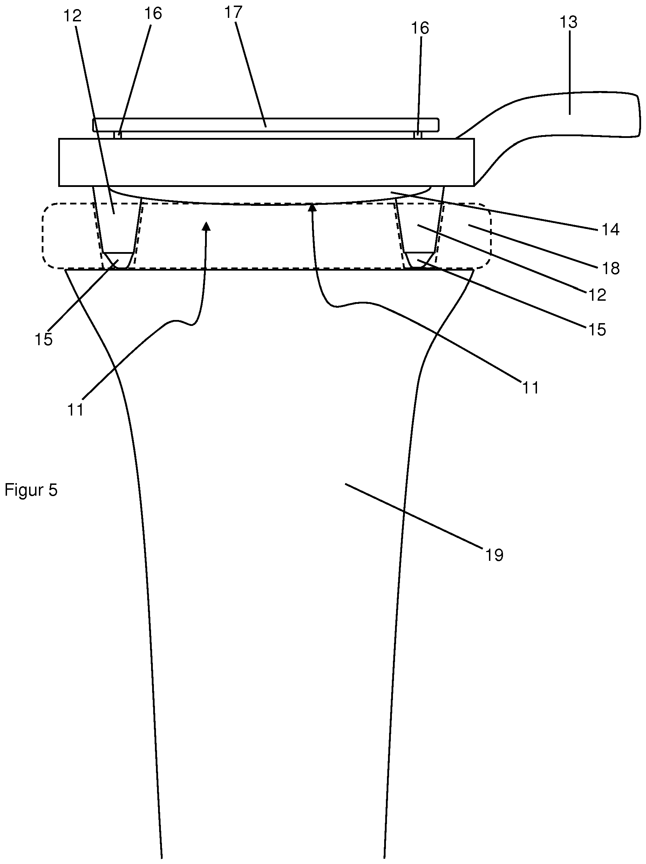

Die Laufflächenformen 14 sollen die nach innen gewölbten tibialen Gleitflächen des Spacers in einem Knochenzement 18 formen. Die tibialen Gleitflächen können dann beispielsweise gegen die Gleitflächen der Kondylen eines Spacers, der mit einer Vorrichtung nach Figur 1 oder 5 geformt wurde, gleiten und dadurch die Funktion eines Kniegelenks nachbilden. Die Dicke des Knochenzements 18 ist durch die auf dem Knochen 19 aufgesetzten Abstandhalter 12 bestimmt.

Wenn der Tibia-Stempel, wie in Figur 4 gezeigt, auf den zähflüssigen (teigigen) Knochenzement 18 aufgepresst ist, wird die Oberfläche des Knochenzements 18 durch die Stempelfläche 11 geformt. Gleichzeitig wird überschüssiger Knochenzement 18 zu den Seiten hin weggedrückt, ohne dass sich Lufteinschlüsse zwischen der Stempelfläche 11 und dem Knochenzement bilden. Nachdem der Knochenzement 18 ausgehärtet ist, wird die Betätigungsleiste 17 bedient, indem sie in Richtung des Tibia-Knochens 19 gedrückt wird. Die Stifte 16 beziehungsweise Stößel 16 drücken dann die gummielastischen Bodenflächen 15 in Richtung des Knochens 19 durch und verformen dabei die Bodenflächen 15. Dadurch werden die Bodenflächen 15 durchgedrückt und der Tibia-Stempel hebt sich von dem gehärteten oder zumindest nicht mehr fließfähigen Knochenzement 18 ab. Diese Situation ist in Figur 5 gezeigt.

Grundsätzlich ist es auch möglich, statt eines gummielastischen Bodens 15 bodenseitige Durchführungen durch die Abstandhalter vorzusehen, so dass sich die Spitzen der Stifte 16 beziehungsweise Stößel 16 direkt auf dem Knochen 19 abstützen und dadurch den Tibia-Stempel von dem Knochenzement 18 abheben. Dazu müssen die Durchführungen aber gut zu den Stiften 16 beziehungsweise Stößeln 16 passen, damit der Knochenzement 18 nicht oder nur in geringem Maße in die Zwischenräume fließen kann, dort aushärtet und eine Bewegung der Stifte 16 beziehungsweise Stößel 16 gegen die Abstandhalter 12 und den gesamten restlichen Stempel unmöglich macht.

Auch kann vorgesehen sein, dass ein größerer Teil der Abstandhalter 12 oder die gesamten Abstandhalter 12 aus einem gummielastischen Material gefertigt ist. Durch die Verformung können sich dann auch die Abstandhalter 12 besser vom Knochenzement lösen. Dafür können sich allerdings Ungenauigkeiten bei der Dicke des erzeugten Spacers ergeben.

Die hier diskutierte Funktion der Stifte 16 beziehungsweise Stößel 16 und die der Abstandhalter 12 kann ohne weiteres und im Rahmen der Erfindung auf Femur-Stempel oder andere erfindungsgemäße Vorrichtungen übertragen werden.

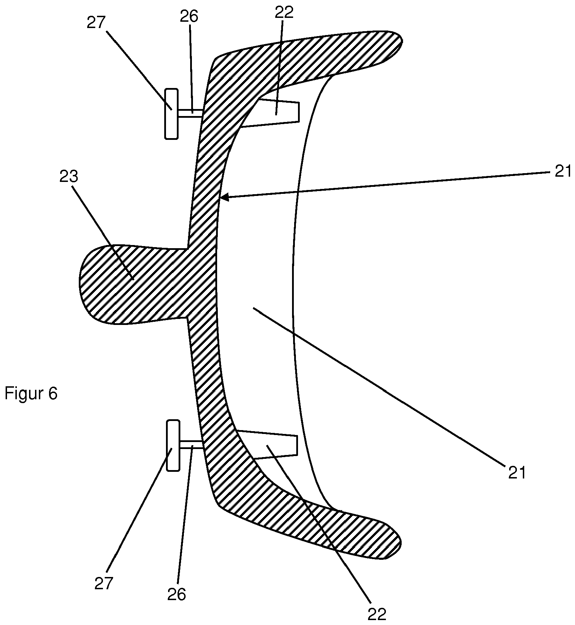

Figur 6 zeigt eine schematische Querschnittansicht eines erfindungsgemäßen Femur-Stempels. Der Femur-Stempel hat eine Stempelfläche 21 zum Formen einer Gleitfläche in einem Knochenzement. Dazu formt der Femur-Stempel die Kondylen-Oberflächen des Knochenzement-Spacers in situ während einer OP auf einem Femur Knochen.

Die Dicke des erzeugten Knochenzement-Spacers wird durch zwei Abstandhalter 22 bestimmt. Auf der der Stempelfläche 21 gegenüberliegenden Seite des Femur-Stempels ist ein Griff 23 angeordnet, mit dem der Femur-Stempel auf einen Knochenzement gedrückt werden kann.

Zwei Schrauben 26 mit Schraubenköpfen 27 sind schraubbar und in Richtung der Schraubenachsen beweglich gegenüber dem restlichen Femur-Stempel in passenden Innengewinden gelagert. Die Schrauben 26 dienen ebenso wie die Stifte 16 beziehungsweise Stößel 16 nach den Figuren 2 bis 5 zur Verformung der Böden der Abstandhalter 22 und damit zum Ablösen des Femur-Stempels vom ausgehärteten Knochenzement-Spacer. Die Stifte 16 beziehungsweise Stößel 16 und die Schrauben 26 haben den Vorteil, dass die Stempel ohne ungünstig wirkende Torsionskräfte oder Drehmomente von dem ausgehärteten oder nicht mehr fließfähigen Knochenzement abgelöst werden können. Werden die Stempel dagegen ohne große Vorsicht mit Hilfe der Griffe 3, 13, 23, beispielsweise durch Hebeln abgelöst, kann es zu einer Beschädigung der gerade erzeugten Artikulationsflächen kommen.

### Bezugszeichenliste

- 1, 11, 21: Stempelfläche
- 2, 12, 22: Abstandhalter
- 3, 13, 23: Griff
- 14: Laufflächenform
- 15: Gummielastischer Boden
- 16: Stift
- 17: Betätigungsleiste
- 18: Knochenzement
- 19: Knochen
- 26: Schraube
- 27: Schraubenkopf

## Patentansprüche

1. Vorrichtung zur in-situ Herstellung von Gelenkspacern aus einem Knochenzement (18) aufweisend eine Stempelfläche (1, 11, 21) zur Ausformung einer Artikulationsoberfläche eines Spacers in dem Knochenzement (18), wobei der Knochenzement (18) auf einer Knochenoberfläche zur Befestigung des Spacers angeordnet ist, wobei die Stempelfläche (1, 11, 21) ein Negativ der zu erzeugenden Artikulationsoberfläche aufweist und die das Negativ bildende Oberfläche zumindest bereichsweise seitlich offen ist, so dass der Knochenzement (18) von der zu formenden Artikulationsoberfläche seitlich wegdrückbar ist, **dadurch gekennzeichnet, dass**
auf der Stempelfläche (1, 11, 21) zumindest ein Abstandhalter (2, 12, 22) zur Auflage auf dem Knochen (19) angeordnet ist, insbesondere zur Auflage auf der Knochenoberfläche angeordnet ist, wobei mit dem Abstandhalter (2, 12, 22) die Dicke des Knochenzements (18) auf der Knochenoberfläche einstellbar ist.

2. Vorrichtung nach Anspruch 1, **dadurch gekennzeichnet, dass**
die das Negativ bildende Oberfläche im Wesentlichen oder vollumfänglich seitlich offen ist, so dass der Knochenzement (18) von der zu formenden Artikulationsoberfläche seitlich in alle Richtungen wegdrückbar ist.

3. Vorrichtung nach einem der vorangehenden Ansprüche, **dadurch gekennzeichnet, dass**
auf der Stempelfläche (1, 11, 21) zumindest zwei Abstandhalter (2, 12, 22) zur Auflage auf dem Knochen (19) angeordnet sind, insbesondere auf der Knochenoberfläche angeordnet ist, mit denen die Dicke des Knochenzements (18) auf der Knochenoberfläche einstellbar ist.

4. Vorrichtung nach Anspruch 3 oder 4, **dadurch gekennzeichnet, dass**
die Höhe der Abstandhalter (2, 12, 22) oder des Abstandhalters (2, 12, 22) über der Stempelfläche (1, 11, 21) variabel einstellbar ist.

5. Vorrichtung nach einem der Ansprüche 3 bis 4 **dadurch gekennzeichnet, dass**
der Abstandhalter (2, 12, 22) oder die Abstandhalter (2, 12, 22) einen durchdrückbaren Boden (15) als Auflage auf dem Kochen (19), insbesondere der Knochenoberfläche aufweist oder aufweisen, wobei sich der durchdrückbare Boden (15) unter Einwirkung einer Kraft in Richtung des Knochens (19), insbesondere der Knochenoberfläche ausbeult und dadurch die Stempelfläche (1, 11, 21) von dem mit der Vorrichtung geformten Knochenzement (18) ablösbar ist, wobei der Boden (15) bevorzugt Gummi-elastisch und die restliche Vorrichtung nicht Gummi-elastisch ist.

6. Vorrichtung nach einem der vorangehenden Ansprüche, **dadurch gekennzeichnet, dass**
die Vorrichtung zumindest eine Ausstoßeinrichtung (16, 26) aufweist, mit der die Stempelfläche (1, 11, 21) von dem mit der Vorrichtung geformten Knochenzement (18) durch Einwirken einer Kraft mechanisch ablösbar ist, wobei bevorzugt die Ausstoßeinrichtung (16, 26) in dem Abstandhalter (2, 12, 22) gelagert ist oder die Ausstoßeinrichtungen (16, 26) in den Abstandhaltern (2, 12, 22) gelagert sind.

7. Vorrichtung nach Anspruch 6, **dadurch gekennzeichnet, dass**
die Ausstoßeinrichtung (16, 26) ein axial beweglicher Stift (16) oder Stößel (16) ist oder eine über ein Gewinde mit der Vorrichtung verbundene Schraube (26) ist, mit dem oder mit der die Vorrichtung auf der Stempelflächenseite vom Knochen (19), insbesondere von der Knochenoberfläche wegdrückbar ist, wobei bevorzugt die Stifte (16), die Stößel (16) und/oder Schrauben (26) mehrerer Ausstoßeinrichtungen (16, 26) derart miteinander verbunden sind,
dass sie sich nur alle gemeinsam auf den Knochen (19), insbesondere die Knochenoberfläche drücken lassen.

8. Vorrichtung nach Anspruch 6 oder 7, **dadurch gekennzeichnet, dass**
die Kraft der Ausstoßeinrichtung (16, 26) senkrecht zur Stempelfläche (1, 11, 21) auf den Knochen (19), insbesondere auf die Knochenoberfläche wirkt.

9. Vorrichtung nach Anspruch 6, 7 oder 8, **dadurch gekennzeichnet, dass**
die Ausstoßeinrichtung (16, 26) in einer Öffnung senkrecht zur Stempelfläche (1, 11, 21) angeordnet ist oder die Ausstoßeinrichtungen (16, 26) in Öffnungen senkrecht zur Stempelfläche (1, 11, 21) angeordnet sind.

10. Vorrichtung nach einem der vorangehenden Ansprüche, **dadurch gekennzeichnet, dass**
an der von der Stempelfläche (1, 11, 21) abgewandten Seite und/oder an der an die Stempelfläche (1, 11, 21) angrenzenden Seitenfläche der Vorrichtung zumindest ein Griff (3, 13, 23) zum Anpressen der Vorrichtung an den Knochen (19) angeordnet ist.

11. Vorrichtung nach einem der vorangehenden Ansprüche, **dadurch gekennzeichnet, dass**
die Vorrichtung im Wesentlichen aus einem thermoplastischen Kunststoff gefertigt ist, bevorzugt aus Polypropylen, Polyethylen, Polytetrafluorethylen, Polyethylenterephthalat, Polybutylenterephathalat, Polyamid-6, Polyamid-6.6, Polyamid-10, Polyamid-12 oder deren Gemischen besteht, wobei vorzugsweise die Vorrichtung gegebenenfalls bis auf den durchdrückbaren Boden (15), Stifte (16), Stößel (16) und/oder Schrauben (26) aus den genannten Materialien besteht.

12. Vorrichtung nach einem der vorangehenden Ansprüche, **dadurch gekennzeichnet, dass**
die Vorrichtung ein Stempel ist, bevorzugt ein Tibia-Stempel zum Formen eines Tibiaplateaus und/oder ein Femur-Stempel zum Formen einer Kondylenoberfläche ist.

13. Stempel nach Aspruch 12 abhängig von Anspruch 6, **dadurch gekennzeichnet, dass**
die Ausstoßeinrichtungen (16, 26) des Tibia-Stempels asymmetrisch zu einer zur Sagitalebene des Patienten parallelen Ebene im Knie angeordnet sind und/oder die Ausstoßeinrichtung (16, 26) oder die Ausstoßeinrichtungen (16, 26) des Femur-Stempels zwischen den Formen für die auszuformenden Kondylen angeordnet ist oder sind, bevorzugt axial zur Femurachse ausgerichtet ist oder sind.

14. Set umfassend zwei Vorrichtungen nach einem der vorangehenden Ansprüche, wobei die erste Vorrichtung zur Ausbildung der Artikulationsoberfläche eines ersten Gelenkspacers vorgesehen ist und die zweite Vorrichtung zur Ausbildung der Artikulationsoberfläche eines zweiten Gelenkspacers vorgesehen ist, wobei die beiden Artikulationsoberflächen im beim Patienten eingesetzten Zustand aneinander anliegen und die Gleitflächen des Gelenkspacers bilden, so dass der Gelenkspacer die Funktion der Knochen (19) des betroffenen Gelenks übernimmt, bevorzugt eines Kniegelenks übernimmt.

## Claims

1. Device for in-situ production of articular spacers from a bone cement (18) comprising a stamp surface (1, 11, 21) for forming an articulation surface of a spacer in the bone cement (18), whereby the bone cement (18) is arranged on a bone surface for attachment of the spacer, whereby the stamp surface (1, 11, 21) comprises a negative image of the articulation surface to be produced and at least regions of the surface forming the negative image are open on the side such that the bone cement (18) can be pushed to the side away from the articulation surface to be formed, **characterised in that**
at least one spacer (2, 12, 22) for support on the bone (19), in particular for support on the bone surface, is arranged on the stamp surface (1, 11, 21), whereby the spacer (2, 12, 22) can be used to adjust the thickness of the bone cement (18) on the bone surface.

2. Device according to claim 1, **characterised in that**
the surface forming the negative image is open on the side, essentially or a long its entire circumference, such that the bone cement (18) can be pushed to the side in all directions away from the articulation surface to be formed.

3. Device according to any one of the preceding claims, **characterised in that**
at least two spacers (2, 12, 22) for support on the bone (19), in particular on the bone surface, are arranged on the stamp surface (1, 11, 21) and can be used to adjust the thickness of the bone cement (18) on the bone surface.

4. Device according to claim 3 or 4, **characterised in that**
the height of the spacers (2, 12, 22) or of the spacer (2, 12, 22) over the stamp surface (1, 11, 21) can be adjusted variably.

5. Device according to any one of the claims 3 to 4, **characterised in that**
the spacer (2, 12, 22) or the spacers (2, 12, 22) comprise a push-through floor (15) for support on the bone (19), in particular the bone surface, whereby the push-through floor (15) bulges outwards when it is exposed to a force in the direction of the bone (19), in particular the bone surface, and, as a result, the stamp surface (1, 11, 21) is detachable from the bone cement (18) formed by means of the device, whereby the floor (15) preferably is rubber-elastic and the remaining device is not rubber-elastic.

6. Device according to any one of the preceding claims, **characterised in that**
the device comprises at least one ejection facility (16, 26) by means of which the stamp surface (1, 11, 21) is mechanically detachable from the bone cement (18) formed by means of the device through the action of a force, whereby the ejection facility (16, 26) preferably is supported, as in a bearing, in the spacer (2, 12, 22) or the ejection facilities (16, 26) are supported, as in a bearing, in the spacers (2, 12, 22).

7. Device according to claim 6, **characterised in that**
the ejection facility (16, 26) is an axially mobile pin (16) or pestle (16) or a screw (26), which is connected to the device by means of a thread, by means of which the device can be pushed away from the bone (19), in particular from the bone surface, on the side of the stamp surface, whereby the pins (16), the pestles (16) and/or screws (26) of multiple ejection facilities (16, 26) are preferably appropriately connected to each other such that they all can only jointly be pushed onto the bone (19), in particular the bone surface.

8. Device according to claim 6 or 7, **characterised in that**
the force of the ejection facility (16, 26) acts on the bone (19), in particular on the bone surface, perpendicular to the stamp surface (1, 11, 21).

9. Device according to claim 6, 7 or 8, **characterised in that**
the ejection facility (16, 26) is arranged in an opening perpendicular to the stamp surface (1, 11, 21) or the ejection facilities (16, 26) are arranged in openings perpendicular to the stamp surface (1, 11, 21).

10. Device according to any one of the preceding claims, **characterised in that**
at least one handle (3, 13, 23) for pressing the device against the bone (19) is arranged on the side facing away from the stamp surface (1, 11, 21) and/or on the lateral surface of the device that is adjacent to the stamp surface (1, 11, 21).

11. Device according to any one of the preceding claims, **characterised in that**
the device is made essentially from a thermoplastic material, preferably from polypropylene, polyethylene, polytetrafluoroethylene, polyethylene terephthalate, polybutylene terephthalate, polyamide-6, polyamide-6.6, polyamide-10, polyamide-12 or mixtures thereof, whereby the device preferably consists of the above-mentioned materials, if applicable except for the push-through floor (15), pins (16), pestles (16) and/or screws (26).

12. Device according to any one of the preceding claims, **characterised in that**
the device is a stamp, preferably a tibial stamp for forming a tibial plateau and/or a femoral stamp for forming a condylar surface.

13. Stamp according to claim 12, dependent on claim 6, **characterised in that**
the ejection facilities (16, 26) of the tibial stamp are arranged asymmetrically with respect to a plane in the knee that is parallel to the sagittal plane of the patient and/or the ejection facility (16, 26) or the ejection facilities (16, 26) of the femoral stamp is or are arranged between the moulds for the condyles to be formed, preferably is or are arranged axial with respect to the femur axis.

14. Set comprising two devices according to any one of the preceding claims, whereby the first device is designed to form the articulation surface of a first articular spacer and the second device is designed to form the articulation surface of a second articular spacer, whereby the two articulation surfaces, in the patient-inserted condition, contact each other and form the gliding surfaces of the articular spacer such that the articular spacer assumes the function of the bones (19) of the respective joint, preferably of a knee joint.

## Revendications

1. Dispositif de fabrication in situ d'espaceurs articulaires à partir d'un ciment osseux (18) présentant une face d'empreinte (1, 11, 21) pour le démoulage d'une surface d'articulation d'un espaceur dans le ciment osseux (18), dans lequel le ciment osseux (18) est disposé sur une surface osseuse pour la fixation de l'espaceur, dans lequel la face d'empreinte (1, 11, 21) présente un négatif de la surface d'articulation à générer et la surface formant le négatif est ouverte latéralement au moins par région de sorte que le ciment osseux (18) peut être écarté latéralement de la surface d'articulation à mouler, **caractérisé en ce que**
au moins un écarteur (2, 12, 22) est disposé sur la face d'empreinte (1, 11, 21) pour l'appui sur l'os (19), est notamment disposé pour l'appui sur la surface osseuse, dans lequel l'épaisseur du ciment osseux (18) sur la surface osseuse peut être réglée avec l'écarteur (2, 12, 22).

2. Dispositif selon la revendication 1, **caractérisé en ce que**
la surface formant le négatif est ouverte latéralement pour l'essentiel ou dans son intégralité de sorte que le ciment osseux (18) peut être écarté latéralement dans tous les sens de la surface d'articulation à mouler.

3. Dispositif selon une des revendications précédentes, **caractérisé en ce que**
au moins deux écarteurs (2, 12, 22), avec lesquels l'épaisseur du ciment osseux (18) sur la surface osseuse peut être réglée, sont disposés sur la face d'empreinte (1, 11, 21) pour l'appui sur l'os (19), sont notamment disposés sur la surface osseuse.

4. Dispositif selon la revendication 3 ou 4, **caractérisé en ce que**
la hauteur des écarteurs (2, 12, 22) ou de l'écarteur (2, 12, 22) au-dessus de la face d'empreinte (1, 11, 21) peut être réglée de manière variable.

5. Dispositif selon une des revendications 3 à 4, **caractérisé en ce que** l'écarteur (2, 12, 22) ou les écarteurs (2, 12, 22) présente ou présentent un fond pouvant être enfoncé (15) comme appui sur l'os (19), notamment la surface osseuse, dans lequel le fond pouvant être enfoncé (15) se gonfle sous l'action d'une force en direction de l'os (19), notamment de la surface osseuse, et la face d'empreinte (1, 11, 21) peut de ce fait être détachée du ciment osseux (18) moulé avec le dispositif, dans lequel le fond (15) est de préférence élastique comme du caoutchouc et le dispositif restant n'est pas élastique comme du caoutchouc.

6. Dispositif selon une des revendications précédentes, **caractérisé en ce que**
le dispositif présente au moins un dispositif d'éjection (16, 26) avec lequel la face d'empreinte (1, 11, 21) peut être détachée mécaniquement du ciment osseux (18) moulé avec le dispositif par l'action d'une force, dans lequel le dispositif d'éjection (16, 26) est de préférence logé dans l'écarteur (2, 12, 22) ou les dispositifs d'éjection (16, 26) sont de préférence logés dans les écarteurs (2, 12, 22).

7. Dispositif selon la revendication 6, **caractérisé en ce que**
le dispositif d'éjection (16, 26) est une tige (16) ou un piston (16) mobile axialement ou est une vis (26) reliée par le biais d'un filetage au dispositif, avec laquelle ou avec lequel le dispositif sur le côté de face d'empreinte peut être écarté de l'os (19), notamment de la surface osseuse, dans lequel les tiges (16), les pistons (16) et/ou vis (26) de plusieurs dispositifs d'éjection (16, 26) sont de préférence relié(e)s entre eux/elles de telle sorte qu'ils/elles ne se laissent pousser que tous collectivement sur l'os (19), notamment la surface osseuse.

8. Dispositif selon la revendication 6 ou 7, **caractérisé en ce que**
la force du dispositif d'éjection (16, 26) agit perpendiculairement à la face d'empreinte (1, 11, 21) sur l'os (19), notamment sur la surface osseuse.

9. Dispositif selon la revendication 6, 7 ou 8, **caractérisé en ce que**
le dispositif d'éjection (16, 26) est disposé dans une ouverture perpendiculairement à la face d'empreinte (1, 11, 21) ou les dispositifs d'éjection (16, 26) sont disposés dans des ouvertures perpendiculairement à la face d'empreinte (1, 11, 21 ).

10. Dispositif selon une des revendications précédentes, **caractérisé en ce que**
au moins une poignée (3, 13, 23) est disposée sur le côté détourné de la face d'empreinte (1, 11, 21) et/ou sur la face latérale du dispositif adjacente à la face d'empreinte (1, 11, 21) pour presser le dispositif contre l'os (19).

11. Dispositif selon une des revendications précédentes, **caractérisé en ce que**
le dispositif est essentiellement fabriqué à partir d'un plastique thermoplastique, se compose de préférence de polypropylène, polyéthylène, polytétrafluoroéthylène, polyéthylène téréphtalate, polybutylène téréphtalate, polyamide-6, polyamide -6.6, polyamide-10, polyamide-12 ou leurs mélanges, dans lequel le dispositif se compose de préférence des matériaux cités éventuellement à l'exception du fond pouvant être enfoncé (15), des tiges (16), des pistons (16) et/ou des vis (26).

12. Dispositif selon une des revendications précédentes, **caractérisé en ce que**
le dispositif est un poinçon, de préférence un poinçon de tibia pour le moulage d'un plateau tibial et/ou un poinçon de fémur pour le moulage d'une surface de condyle.

13. Poinçon selon la revendication 12 dépendant de la revendication 6, **caractérisé en ce que**
les dispositifs d'éjection (16, 26) du poinçon de tibia sont disposés de manière asymétrique par rapport à un plan dans le genou parallèle au plan sagittal du patient et/ou le dispositif d'éjection (16, 26) ou les dispositifs d'éjection (16, 26) du poinçon de fémur est ou sont disposé(s) entre les moules pour les condyles à démouler, est ou sont de préférence orienté(s) axialement par rapport à l'axe fémoral.

14. Ensemble comprenant deux dispositifs selon une des revendications précédentes, dans lequel le premier dispositif est prévu pour la réalisation de la surface d'articulation d'un premier espaceur articulaire et le second dispositif est prévu pour la réalisation de la surface d'articulation d'un second espaceur articulaire, dans lequel les deux surfaces d'articulation reposent l'une sur l'autre dans l'état utilisé chez le patient et forment les faces de glissement de l'espaceur articulaire de sorte que l'espaceur articulaire prend la fonction des os (19) de l'articulation concernée, de préférence d'une articulation du genou.
